# EUROPEAN PATENT APPLICATION

(11) **EP 2 921 229 A1**
(43) Date of publication of application: **23.09.2015**
(21) Application number: 13855478.7
(22) Date of filing: 14.11.2013
(51) Int. Cl.: B01J 31/22, C07B 53/00, C07C 29/56, C07C 35/17, C07B 61/00, C07C 39/06, C07C 39/17, C07C 43/23, C07F 5/06

(54) **ALUMINUM CATALYST**

(30) Priority: 15.11.2012 JP 2012251301
(71) Applicant: Takasago International Corporation, Tokyo 144-8721 (JP)
(72) Inventor: ITOH, Hisanori, Hiratsuka-shi, Kanagawa 254-0073 (JP); HORI, Yoji, Hiratsuka-shi, Kanagawa 254-0073 (JP)
(74) Representative: Takeuchi, Maya
(86) International application number: PCT/JP2013/080799
(87) International publication number: WO 2014/077321

(57) **Abstract**

The present invention makes it possible to obtain 1-isopulegol with high yield and high selectivity by performing a citronellal selective-ring-closing reaction using an aluminum catalyst obtained by reacting: one or more types of aluminum compound selected from aluminum alkyls and aluminum hydrides; and one or more types of phenol compounds selected from 2-cycloalkyl-6-arylphenols and di(2-cycloalkyl-6-arylphenols).

## Description

### Technical Field

The present invention relates to an organoaluminum compound obtained by reacting at least one aluminum compound selected from an alkylaluminum and a hydridoaluminum with at least one of phenol compounds selected from a 2-cycloalkyl-6-arylphenol and a di(2-cycloalkyl-6-arylphenol).

The present invention further relates to a method for producing isopulegol by cyclizing citronellal with high n-selectivity using the organoaluminum compound as a catalyst.

### Background Art

Conventionally, menthol, particularly L-menthol, is very important as flavor or fragrance having fresh-feeling and its use is very wide. As synthesis methods of L-menthol, a method of obtaining it by optical resolution of DL-menthol and a method of obtaining L-menthol by an asymmetric synthesis method are known (Non-Patent Document 1). In the production step of L-menthol by the asymmetric synthesis method, L-menthol is obtained by hydrogenating L-isopulegol which is a precursor. However, in order to synthesize the L-isopulegol, a high selective cyclization reaction of D-citronellal is an important step.

As the selective cyclization reaction of D-citronellal, the disclosed method, that is, the production of L-isopulegol using zinc bromide as a catalyst, was already performed (Patent Document 1). In this case, the ratio of L-isopulegol to other isomers is about 90% as diastereoselectivity.

A selective cyclization reaction of citronellal by an aluminum siloxide catalyst has been reported (Patent Documents 2 and 3). In this case, the diastereoselectivity of isopulegol formed is up to 96%.

Selective cyclization reactions of D-citronellal by tris(2,6-diarylphenoxy)aluminum and its similar catalyst have been reported (Patent Documents 4 to 7). In these Patent Documents, the diastereoselectivity of L-isopulegol formed is 99% or more in some cases.

Furthermore, selective cyclization reactions by other aluminum catalysts have been reported (Patent Documents 8 to 11). In these Patent Documents, the diastereoselectivity of isopulegol is up to 98%.

### Prior Art Documents

### Patent Documents

Patent Document 1: JP-B-59-45661
Patent Document 2: JP-T-2009-510005 (the term "JP-T" used herein means a published Japanese translation of a PCT patent application)
Patent Document 3: JP-T-2009-510006
Patent Document 4: JP-A-2002-212121
Patent Document 5: DE-A-102005023953
Patent Document 6: JP-T-2008-524287
Patent Document 7: JP-T-2008-538101
Patent Document 8: WO2009/144906
Patent Document 9: JP-T-2012-512135
Patent Document 10: JP-T-2012-512136
Patent Document 11: JP-A-2011-246366

### Summary of the Invention

### Problems that the Invention is to Solve

However, in the methods of Patent Documents 1 to 3 and Patent Documents 8 to 11, the diastereoselectivity of isopulegol is very high but is not so satisfactory and there is room for improvement. Moreover, the conventionally used (2,6-diarylphenoxy)aluminum compound catalyst is expensive.

Patent Documents 4 to 7 describes diastereoselective cyclization reactions of citronellal using a tris(2,6-diarylphenoxy)aluminum compound as a catalyst and, in Patent Document 4, it is clearly described in Comparative Examples in the text of the specification that the tris(2,6-dialkylphenoxy)aluminum compound does not result in high selectivity.

Moreover, in Patent Document 6, there is a description of alkyl groups having from 1 to 8 carbon atoms which are each independently present at the 2-position and the 6-position in claim 1 but all Examples relates to diastereoselective cyclization reactions of citronellal using only tris(2,6-diphenylphenoxy)aluminum catalyst and there is no description in claim 1 and the specification that the 2-position and/or the 6-position of the phenol is a cyclic alkyl group.

Furthermore, Patent Document 7 describes a diastereoselective cyclization reaction of citronellal using a {bis(2,6-diarylphenoxy)aluminum compound as a catalyst and the ligand of the catalyst is limited to a bis(2,6-diarylphenol).

An object of the present invention relates to development of a catalyst for obtaining isopulegol with high diastereoselectivity through high selective cyclization reaction of citronellal. In addition, it relates to a method for producing L-isopulegol which is an important synthesis precursor of L-menthol and is useful as a material of flavor or fragrance, particularly obtained by high selective cyclization reaction of D-citronellal using the catalyst.

### Means for Solving the Problems

As a result of intensive investigations to solve the above problems, the present inventors have succeeded in obtaining an aluminum catalyst, which has hitherto not been reported, using a 2-cycloalkyl-6-arylphenol or a bis(2-cycloalkyl-6-phenyl)phenol, which is relatively inexpensive and easily producible, as a ligand.

They have further found that, when the cyclization reaction of citronellal is performed by using the aluminum catalyst, between four kinds of isomers, i.e. isopulegol, isoisopulegol, neoisopulegol and neoisoisopulegol, isopulegol is obtained with high selectivity of 97% or more in terms of isomer ratio with high yield, and thus, the present invention has been accomplished.

That is, the present invention includes each of the following inventions.
<1> An aluminum catalyst obtained by reacting at least one compound of an alkylaluminum compound represented by the following general formula (1) and a hydridoaluminum compound represented by the following general formula (2) with a hydroxy compound represented by the following general formula (3):

   AlHₘ(Lg)₃₋ₘ (1)

   wherein Al is aluminum; Lg is a branched, linear or cyclic alkyl group having from 1 to 8 carbon atoms; and m is an integer of 0 to 3;

   MAlH₄ (2)

   wherein Al is aluminum; and M is lithium, sodium or potassium; 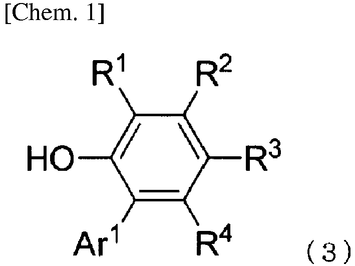 wherein Ar¹ is an aryl group having from 6 to 15 carbon atoms, which may have a substituent, or a heteroaryl group having from 2 to 15 carbon atoms, which may have a substituent;
   R¹ is a cyclic alkyl group having from 5 to 12 carbon atoms, which may have a substituent; R², R³ and R⁴ each independently are a hydrogen atom, an alkyl group having from 1 to 8 carbon atoms, a cyclic alkyl group having from 5 to 12 carbon atoms, a perfluoroalkyl group having from 1 to 4 carbon atoms, an alkoxy group having from 1 to 8 carbon atoms, an aralkyl group having from 7 to 12 carbon atoms, which may have a substituent, a halogen atom, an organosilyl group, an aryl group having from 6 to 15 carbon atoms, which may have a substituent, a dialkylamino group having from 2 to 8 carbon atoms, a thioalkoxy group having from 1 to 4 carbon atoms, a nitro group or a polymer chain; and R² and R³, or R³ and R⁴ may each independently be combined with each other to form a condensed benzene ring, a condensed substituted-benzene ring, a trimethylene group, a tetramethylene group, a pentamethylene group, a methylenedioxy group, an ethylenedioxy group or a trimethylenedioxy group.
<2> An aluminum catalyst obtained by reacting at least one compound of an alkylaluminum compound represented by the following general formula (1) and a hydridoaluminum compound represented by the following general formula (2) with a hydroxy compound represented by the following general formula (4):

   AlHₘ(Lg)₃₋ₘ (1)

   wherein Al is aluminum; Lg is a branched, linear or cyclic alkyl group having from 1 to 8 carbon atoms; and m is an integer of 0 to 3;

   MAlH₄ (2)

   wherein Al is aluminum; and M is lithium, sodium or potassium; 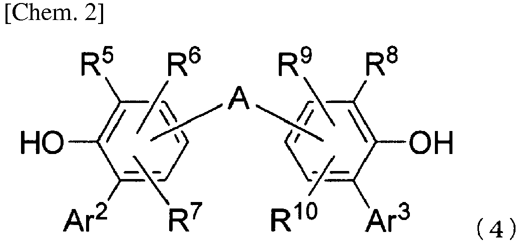 wherein Ar² and Ar³ each independently are an aryl group having from 6 to 15 carbon atoms, which may have a substituent, or a heteroaryl group having from 2 to 15 carbon atoms, which may have a substituent;
   R⁵ and R⁸ each independently are a cyclic alkyl group having from 5 to 12 carbon atoms, which may have a substituent; R⁶, R⁷, R⁹ and R¹⁰ each independently are a hydrogen atom, an alkyl group having from 1 to 8 carbon atoms, a cyclic alkyl group having from 5 to 12 carbon atoms, a perfluoroalkyl group having from 1 to 4 carbon atoms, an alkoxy group having from 1 to 8 carbon atoms, an aralkyl group having from 7 to 12 carbon atoms, which may have a substituent, a halogen atom, an organosilyl group, an aryl group having from 6 to 15 carbon atoms, which may have a substituent, a dialkylamino group having from 2 to 8 carbon atoms, a thioalkoxy group having from 1 to 4 carbon atoms, a nitro group or a polymer chain; and R⁶ and R⁷, or R⁹ and R¹⁰ may each independently be combined with each other to form a condensed benzene ring, a condensed substituted-benzene ring, a trimethylene group, a tetramethylene group, a pentamethylene group, a methylenedioxy group, an ethylenedioxy group or a trimethylenedioxy group; and one or more of R⁶ or R⁷, and R⁹ or R¹⁰ may be combined with A to form an aromatic ring or a non-aromatic ring;
   A is (i) a single bond, (ii) a linear, branched or cyclic alkylene group having from 1 to 25 carbon atoms, which may have one or more of a substituent and an unsaturated bond, (iii) an arylene group having from 6 to 15 carbon atoms, which may have a substituent, (iv) a heteroarylene group having from 2 to 15 carbon atoms, which may have a substituent, or (v) a functional group or a hetero element, which is selected from the group consisting of -O-, -S-, -N(R¹¹)-, -S(O)-, -C(O)-, -S(O)₂-, -P(R¹¹)-, -(R¹¹)P(O)- and -Si(R¹²R¹³)-, wherein R¹¹ to R¹³ each independently are an alkyl group having from 1 to 6 carbon atoms, a cyclic alkyl group having from 5 to 8 carbon atoms, an aralkyl group having from 7 to 12 carbon atoms, which may have a substituent, or an aryl group having from 6 to 10 carbon atoms, which may have a substituent.
<3> A method for producing isopulegol represented by the general formula (6), comprising a step of selectively cyclizing citronellal represented by the general formula (5) using the aluminum catalyst according to the above <1> or the aluminum catalyst according to the above <2>. 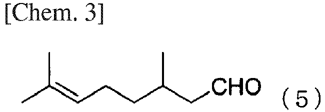 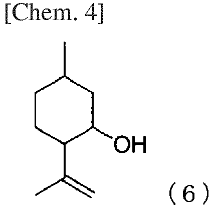
<4> A method for producing optically active isopulegol represented by the general formula (8), comprising a step of selectively cyclizing citronellal represented by the general formula (7) using the aluminum catalyst according to the above <1> or the aluminum catalyst according to the above <2>. 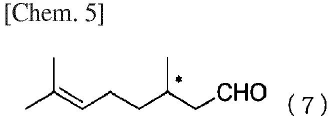 wherein * means an asymmetric carbon atom; 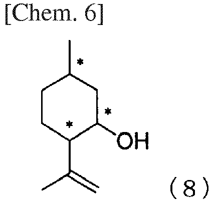 wherein * means an asymmetric carbon atom.
<5> A method for producing isopulegol represented by the general formula (6), comprising a step of selectively cyclizing citronellal represented by the general formula (5) in the presence of at least one compound of the following compounds I and II using the aluminum catalyst according to the above <1> or the aluminum catalyst according to the above <2>:
   I. at least one acid;
   II. at least one compound selected from the group consisting of an aldehyde other than citronellal, an acid anhydride, a ketone, an acid halide, an epoxy compound and a vinyl ether. 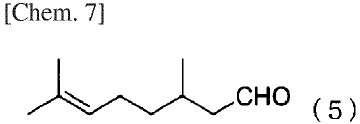 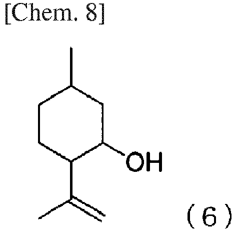
<6> A method for producing optically active isopulegol represented by the general formula (8), comprising a step of selectively cyclizing citronellal represented by the general formula (7) in the presence of at least one compound of the following compounds I and II using the aluminum catalyst according to the above <1> or the aluminum catalyst according to the above <2>:
   I. at least one acid;
   II. at least one compound selected from the group consisting of an aldehyde other than citronellal, an acid anhydride, a ketone, an acid halide, an epoxy compound and a vinyl ether. 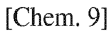 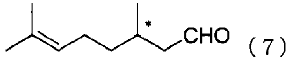
   wherein * means an asymmetric carbon atom; 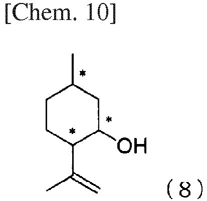 wherein * means an asymmetric carbon atom.

### Advantage of the Invention

As described above, the present invention provides an aluminum catalyst obtained by reacting at least one of a specific alkylaluminum and hydridoaluminum with a specific hydroxy compound selected from a 2-cycloalkyl-6-arylphenol or a di(2-cycloalkyl-6-arylphenol). Use of the aluminum catalyst can give very high diastereoselectivity in a selective cyclization reaction of citronellal. Furthermore, by filtering a reaction solution, the catalyst can be reutilized, which is industrially advantageous. The ligand after catalyst deactivation can again be reutilized as the catalyst through recovery.

The present invention can further provide a method for producing isopulegol which is useful as a material of flavor or fragrance and is an important synthesis intermediate of menthol, by using the aluminum catalyst with high yield and high selectivity.

### Brief Description of the Drawings

FIG. 1 is a view showing ¹H-NMR spectrum of a solid obtained by reacting 2-cyclohexyl-6-phenylphenol with triethylaluminum in Example 1.
FIG. 2 is an enlarged view at low magnetic field side of ¹H-NMR spectrum shown in FIG. 1.
FIG. 3 is a view showing ¹H-NMR spectrum of 2-cyclohexyl-6-phenylphenol (CPP).
FIG. 4 is an enlarged view at low magnetic field side of ¹H-NMR spectrum shown in FIG. 3.
FIG. 5 is a graph showing the results of reaction conversion in the synthesis of d-isopulegol.

### Modes for Carrying Out the Invention

The present invention is described in detail below.

The aluminum compound to be used for producing the aluminum catalyst in the present invention is at least one aluminum compound selected from an alkylaluminum represented by the general formula (1) and a hydridoaluminum represented by the general formula (2).

AlHₘ(Lg)₃₋ₘ (1)

In the general formula (1), Al is aluminum; Lg is a branched, linear or cyclic alkyl group having from 1 to 8 carbon atoms; and m is an integer of 0 to 3.

In the general formula (1), examples of the alkyl group represented by Lg include methyl group, ethyl group, n-propyl group, isopropyl group, n-butyl group, isobutyl group, sec-butyl group, tert-butyl group, pentyl group, hexyl group, heptyl group, octyl group, cyclopentyl group, cyclohexyl group, cycloheptyl group and cyclooctyl group.

MAlH₄ (2)

In the general formula (2), Al is aluminum and M is lithium, sodium, or potassium.

The hydroxy compound to be used for producing the aluminum catalyst in the present invention is at least one hydroxy compound selected from the group consisting of a 2-cycloalkyl-6-arylphenol represented by the following general formula (3) and a di(2-cycloalkyl-6-arylphenol) represented by the general formula (4).

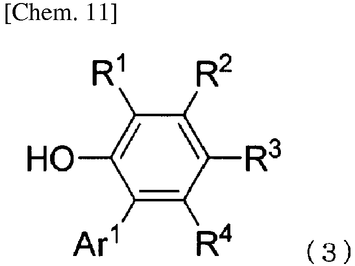

In the general formula (3), Ar¹ is an aryl group having from 6 to 15 carbon atoms, which may have a substituent, or a heteroaryl group having from 2 to 15 carbon atoms, which may have a substituent. R¹ is a cyclic alkyl group having from 5 to 12 carbon atoms, which may have a substituent; R², R³ and R⁴ each independently are a hydrogen atom, an alkyl group having from 1 to 8 carbon atoms, a cyclic alkyl group having from 5 to 12 carbon atoms, a perfluoroalkyl group having from 1 to 4 carbon atoms, an alkoxy group having from 1 to 8 carbon atoms, an aralkyl group having from 7 to 12 carbon atoms, which may have a substituent, a halogen atom, an organosilyl group, an aryl group having from 6 to 15 carbon atoms, which may have a substituent, a dialkylamino group having from 2 to 8 carbon atoms, a thioalkoxy group having from 1 to 4 carbon atoms, a nitro group or a polymer chain; and R² and R³, or R³ and R⁴ may each independently be combined with each other to form a condensed benzene ring, a condensed substituted-benzene ring, a trimethylene group, a tetramethylene group, a pentamethylene group, a methylenedioxy group, an ethylenedioxy group or a trimethylenedioxy group.

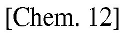

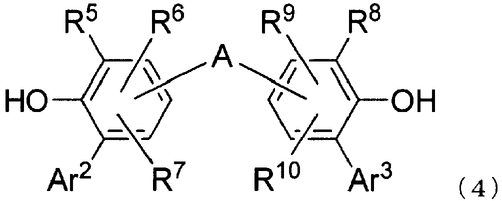

In the general formula (4), Ar² and Ar³ each independently are an aryl group having from 6 to 15 carbon atoms, which may have a substituent, or a heteroaryl group having from 2 to 15 carbon atoms, which may have a substituent. R⁵ and R⁸ each independently are a cyclic alkyl group having from 5 to 12 carbon atoms, which may have a substituent; R⁶, R⁷, R⁹ and R¹⁰ each independently are a hydrogen atom, an alkyl group having from 1 to 8 carbon atoms, a cyclic alkyl group having from 5 to 12 carbon atoms, a perfluoroalkyl group having from 1 to 4 carbon atoms, an alkoxy group having from 1 to 8 carbon atoms, an aralkyl group having from 7 to 12 carbon atoms, which may have a substituent, a halogen atom, an organosilyl group, an aryl group having from 6 to 15 carbon atoms, which may have a substituent, a dialkylamino group having from 2 to 8 carbon atoms, a thioalkoxy group having from 1 to 4 carbon atoms, a nitro group or a polymer chain; and R⁶ and R⁷, or R⁹ and R¹⁰ may each independently be combined with each other to form a condensed benzene ring, a condensed substituted-benzene ring, a trimethylene group, a tetramethylene group, a pentamethylene group, a methylenedioxy group, an ethylenedioxy group or a trimethylenedioxy group; and one or more of R⁶ or R⁷, and R⁹ or R¹⁰ may be combined with A to form an aromatic ring or a non-aromatic ring.

A is (i) a single bond, (ii) a linear, branched or cyclic alkylene group having from 1 to 25 carbon atoms, which may have one or more of a substituent and an unsaturated bond, (iii) an arylene group having from 6 to 15 carbon atoms, which may have a substituent, (iv) a heteroarylene group having from 2 to 15 carbon atoms, which may have a substituent, or (v) a functional group or a hetero element, which is selected from the group consisting of -O-, -S-, -N(R¹¹)-, -S(O)-, -C(O)-, -S(O)₂-, -P(R¹¹)-, -(R¹¹)P(O)- and -Si(R¹²R¹³)-, wherein R¹¹ to R¹³ each independently are an alkyl group having from 1 to 6 carbon atoms, a cyclic alkyl group having from 5 to 8 carbon atoms, an aralkyl group having from 7 to 12 carbon atoms, which may have a substituent, or an aryl group having from 6 to 10 carbon atoms, which may have a substituent.

As the specific functional groups in the hydroxy compounds represented by the above general formulae (3) and (4), the following examples are exemplified.

Examples of the aryl group having from 6 to 15 carbon atoms, which is represented by Ar¹, Ar² and Ar³, include phenyl group, α-naphthyl group, β-naphthyl group, and the like. The aryl group having from 6 to 15 carbon atoms may have a substituent to be mentioned later.

Examples of the heteroaryl group having from 2 to 15 carbon atoms, which is represented by Ar¹, Ar² and Ar³, include furyl group, thienyl group, pyronyl group, benzofuryl group, isobenzofuryl group, benzothienyl group, indolyl group, isoindolyl group, carbazoyl group, pyridyl group, quinolyl group, isoquinolyl group, pyrazyl group, ferrocenyl group, and the like. The heteroaryl group having from 2 to 15 carbon atoms may have a substituent to be mentioned later.

Here, examples of the substituent in Ar¹, Ar² and Ar³ include an alkyl group having from 1 to 6 carbon atoms such as methyl group, ethyl group, n-propyl group, isopropyl group, n-butyl group, isobutyl group, sec-butyl group, tert-butyl group, pentyl group and hexyl group; a cyclic alkyl group having from 5 to 12 carbon atoms such as cyclopentyl group, cyclohexyl group, cycloheptyl group and cyclooctyl group; a perfluoroalkyl group having from 1 to 4 carbon atoms such as trifluoromethyl group, pentafluoroethyl group, heptafluoropropyl group and nonafluorobutyl group; an alkoxy group having from 1 to 4 carbon atoms such as methoxy group, ethoxy group, n-propoxyl group, isopropoxy group, n-butoxy group, isobutoxy group, sec-butoxy group and tert-butoxy group; a halogen atom such as fluorine atom, chlorine atom, bromine atom and iodine atom; an aralkyl group having from 7 to 12 carbon atoms such as benzyl group, phenylethyl group and naphthylmethyl group; a tri-(C₁₋₆)alkylsilyl group such as trimethylsilyl group, triethylsilyl group, triisopropylsilyl group, dimethylisopropylsilyl group, diethylisopropylsilyl group, dimethyl(2,3-dimethyl-2-butyl)silyl group, tert-butyldimethylsilyl group and dimethylhexylsilyl group; a dialkylamino group having from 2 to 8 carbon atoms such as dimethylamino group, diethylamino group, dibutylamino group and the like. Further examples thereof include polymer chains such as 6,6-nylon chain, vinyl polymer chain and styrene polymer chain.

Examples of the cyclic alkyl group having from 5 to 12 carbon atoms, which is represented by R¹, R⁵ and R⁸, include cyclopentyl group, cyclohexyl group, cycloheptyl group, cyclooctyl group, cyclodecyl group, cyclododecyl group, norbornyl group, tricyclo[6.2.1.0^{2,7}]-4-undecyl group, and the like. The cyclic alkyl group having from 5 to 12 carbon atoms may have a substituent and examples of the substituent include the same substituents as those exemplified as the substituents in Ar¹, Ar² and Ar³.

Examples of the alkyl group having from 1 to 8 carbon atoms, which is represented by R², R³, R⁴, R⁶, R⁷, R⁹ and R¹⁰, include methyl group, ethyl group, n-propyl group, isopropyl group, n-butyl group, isobutyl group, sec-butyl group, tert-butyl group, pentyl group, hexyl group, heptyl group, octyl group, and the like.

Examples of the cyclic alkyl group having from 5 to 12 carbon atoms, which is represented by R², R³, R⁴, R⁶, R⁷, R⁹ and R¹⁰, include cyclopentyl group, cyclohexyl group, cycloheptyl group, cyclooctyl group, cyclodecyl group, cyclododecyl group, norbornyl group, tricyclo[6.2.1.0^{2,7}]-4-undecyl group, and the like.

Examples of the perfluoroalkyl group having from 1 to 4 carbon atoms, which is represented by R², R³, R⁴, R⁶, R⁷, R⁹ and R¹⁰, include trifluoromethyl group, pentafluoroethyl group, heptafluoropropyl group, nonafluorobutyl group, and the like.

Examples of the alkoxy group having from 1 to 8 carbon atoms, which is represented by R², R³, R⁴, R⁶, R⁷, R⁹ and R¹⁰, include methoxy group, ethoxy group, n-propoxyl group, isopropoxy group, n-butoxy group, isobutoxy group, sec-butoxy group, tert-butoxy group, pentoxy group, hexoxy group, heptoxy group, octoxy group, and the like.

Examples of the aralkyl group having from 7 to 12 carbon atoms, which is represented by R², R³, R⁴, R⁶, R⁷, R⁹ and R¹⁰, include benzyl group, 1-phenylethyl group, 2-phenylethyl group, α-naphthylmethyl group, β-naphthylmethyl group, and the like. The aralkyl group having from 7 to 12 carbon atoms may have a substituent and examples of the substituent include the same substituents as those exemplified as the substituents in Ar¹, Ar² and Ar³.

Examples of the halogen atom, which is represented by R², R³, R⁴, R⁶, R⁷, R⁹ and R¹⁰, include fluorine atom, chlorine atom, bromine atom, iodine atom, and the like.

Examples of the organosilyl group, which is represented by R², R³, R⁴, R⁶, R⁷, R⁹ and R¹⁰, include tri-substituted silyl groups. The substituents of the tri-substituted ones are three substituents selected from alkyl groups having from 1 to 6 carbon atoms, aryl groups having from 6 to 18 carbon atoms and aralkylsilyl groups having from 7 to 19 carbon atoms, and those may be the same or different.

Examples of the alkyl group having from 1 to 6 carbon atoms include methyl group, ethyl group, isopropyl group, 2,3-dimethyl-2-butyl group, hexyl group and tert-butyl group.

Examples of the aryl group having from 6 to 18 carbon atoms include phenyl group and naphthyl group.

Examples of the aralkyl group having from 7 to 19 carbon atoms include benzyl group and p-xylyl group.

Examples of the organosilyl group include tri-substituted silyl groups, for example, tri-C₁₋₆ alkylsilyl groups such as trimethylsilyl group, triethylsilyl group, triisopropylsilyl group, dimethylisopropylsilyl group, diethylisopropylsilyl group, dimethyl(2,3-dimethyl-2-butyl)silyl group, tert-butyldimethylsilyl group and dimethylhexylsilyl group; di-C₁₋₆ alkyl-C₆₋₁₈ arylsilyl groups such as dimethylcumylsilyl group; di-C₆₋₁₈ aryl-C₁₋₆ alkylsilyl groups such as tert-butyldiphenylsilyl group and diphenylmethylsilyl group; tri-C₆₋₁₈ arylsilyl groups such as triphenylsilyl group; tri-C₇₋₁₉ aralkylsilyl group such as tribenzylsilyl group and tri-p-xylylsilyl group, and the like.

Examples of the aryl group having from 6 to 15 carbon atoms, which is represented by R², R³, R⁴, R⁶, R⁷, R⁹ and R¹⁰, include phenyl group, naphthyl group, phenanthryl group and anthranyl group. The aryl group having from 6 to 15 carbon atoms may have a substituent and examples of the substituent include the same substituents as those exemplified as the substituents in Ar¹, Ar² and Ar³.

Examples of the dialkylamino group having from 2 to 8 carbon atoms, which is represented by R², R³, R⁴, R⁶, R⁷, R⁹ and R¹⁰, include dimethylamino group, diethylamine group, dipropylamino group, diisopropylamino group, dibutylamino group, and the like.

Examples of the thioalkoxy group having from 1 to 4 carbon atoms, which is represented by R², R³, R⁴, R⁶, R⁷, R⁹ and R¹⁰, include methylthio group, ethylthio group, n-propylthio group, isopropylthio group, n-butylthio group, isobutylthio group, sec-butylthio group and tert-butylthio group.

Examples of the polymer chain which is represented by R², R³, R⁴, R⁶, R⁷, R⁹ and R¹⁰ include 6,6-nylon chain, vinyl polymer chain, styrene polymer chain, and the like.

Specific examples of the hydroxy compound represented by the general formula (3) include the following structures.

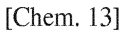

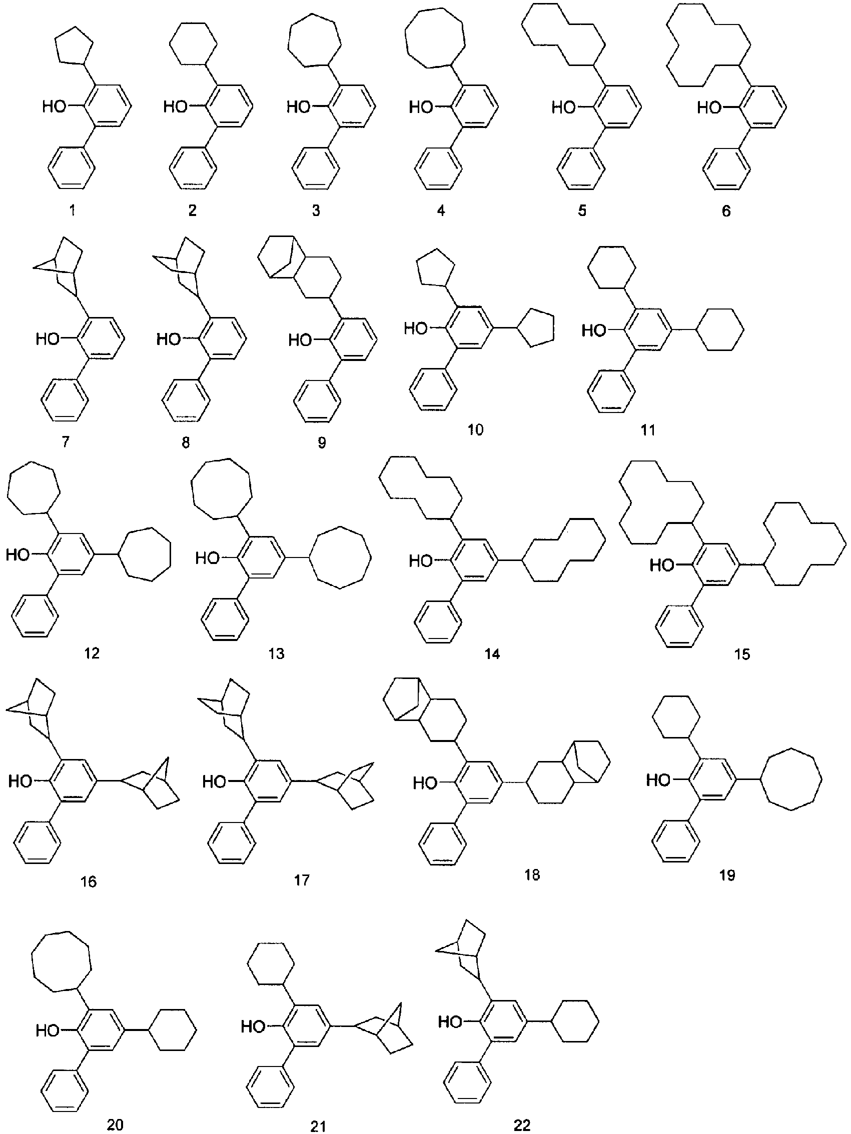

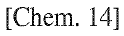

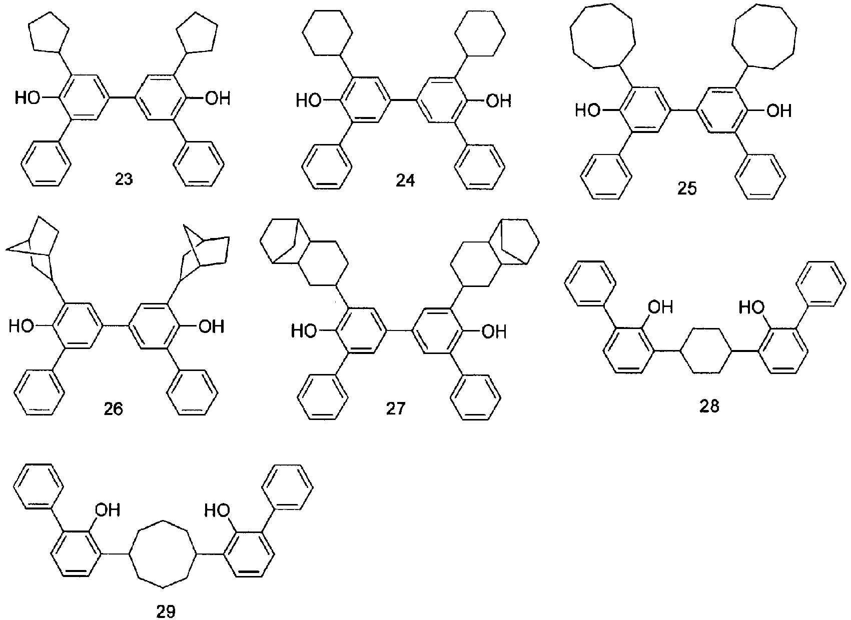

In the general formula (4), examples of (ii) the linear, branched or cyclic alkylene group having from 1 to 25 carbon atoms, which is represented by A, include methylene group, ethylene group, isopropylene group, n-butylene group, isobutylene group, sec-butylene group, tert-butylene group, dodecylene group, undecylene group, cyclopentylene group, cyclohexylene group, cycloheptylene group, cyclooctylene group, cyclodecylene group, cyclododecylene group, norbornylene group and tricyclo[6.2.1.0^{2,7}]-4-undecylene group.

The linear, branched or cyclic alkylene group having from 1 to 25 carbon atoms may have one or more of a substituent and an unsaturated bond. Examples of the substituent include the same substituents as those exemplified as the substituents in the above Ar¹, Ar² and Ar³.

Examples of the (iii) arylene group having from 6 to 15 carbon atoms in the general formula (4), which is represented by A, include phenylene group, naphthylene group, anthracenylene group, and the like. The arylene group having from 6 to 15 carbon atoms may have a substituent and examples of the substituent include the same substituents as the substituents exemplified in the above Ar¹, Ar² and Ar³.

Examples of the (iv) heteroarylene group having from 2 to 15 carbon atoms in the general formula (4), which is represented by A, include furylene group, thienylene group, pyronylene group, benzofurylene group, isobenzofurylene group, benzothienylene group, indolylene group, isoindolylene group, carbazoylene group, pyridylene group, quinolylene group, isoquinolylene group, pyrazylene group, ferrocenylene group, and the like. The heteroarylene group having from 2 to 15 carbon atoms may have a substituent and examples of the substituent include the same substituents as those exemplified as the substituents in the above Ar¹, Ar² and Ar³.

In the general formula (4), A may be (v) a functional group or a hetero element, which is selected from the group consisting of -O-, -S-, -N(R¹¹)-, -S(O)-, -C(O)-, -S(O)₂-, -P(R¹¹)-, -(R¹¹)P(O)- and -Si(R¹²R¹³)-. Here, R¹¹ to R¹³ each independently are one or more groups of an alkyl group having from 1 to 6 carbon atoms, a cyclic alkyl group having from 5 to 8 carbon atoms, an aralkyl group having from 7 to 12 carbon atoms, which may have a substituent, and an aryl group having from 6 to 10 carbon atoms, which may have a substituent. Examples of the substituent include the same substituents as those exemplified as the substituents in the above Ar¹, Ar² and Ar³. As A, -O-, -S-, -S(O)-, -S(O)2- or -Si(R¹²R¹³)- is preferable.

Specific examples of A include the following structures. The wave line shows a bonding site to the remaining sites of each ligand structure so as to fall within the range disclosed in the present specification.

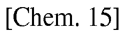

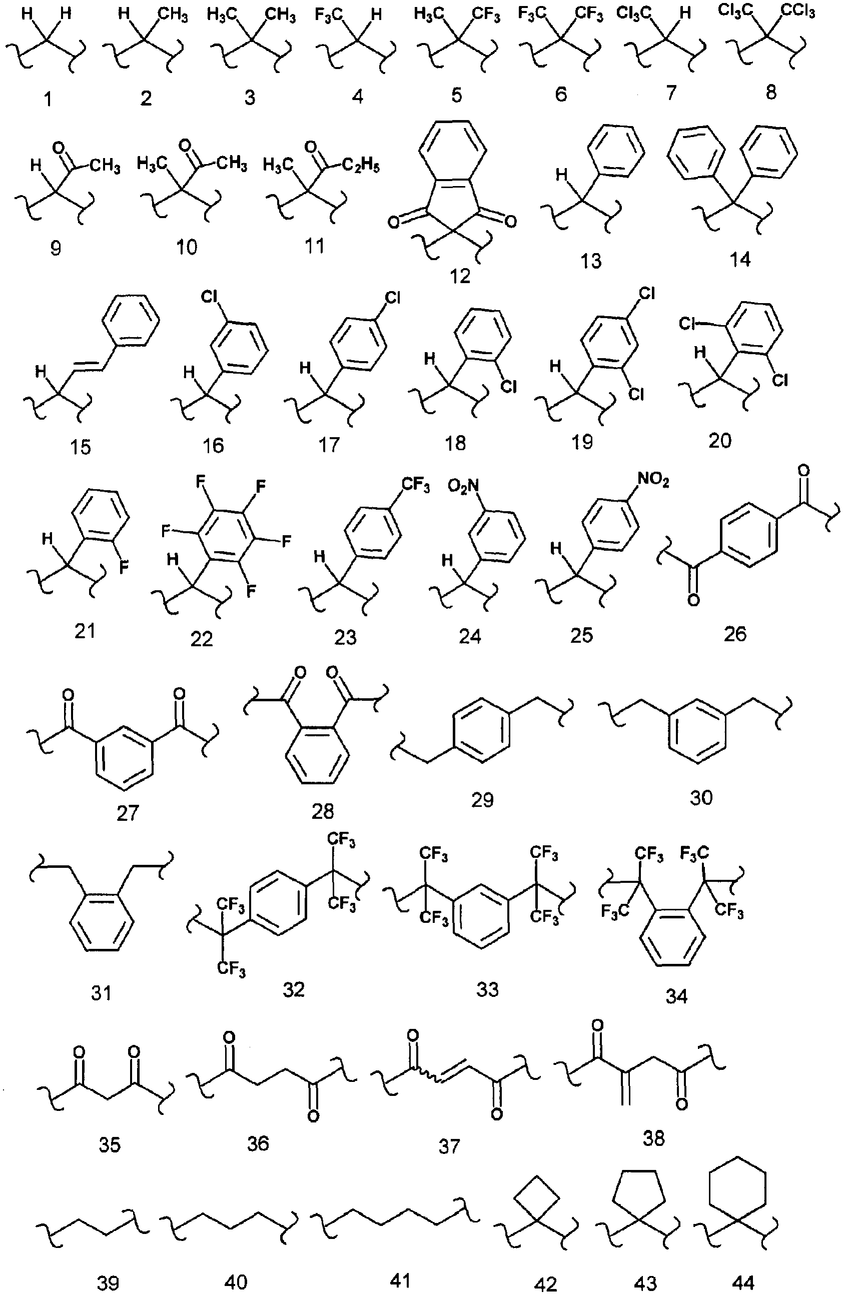

The structures 1 to 44 shown in the above may have a substituent and examples of the substituent include the same substituents as those exemplified in the aryl group having from 6 to 15 carbon atoms in the above Ar¹.

Specific examples of the hydroxy compound represented by the general formula (4) include the following structures.

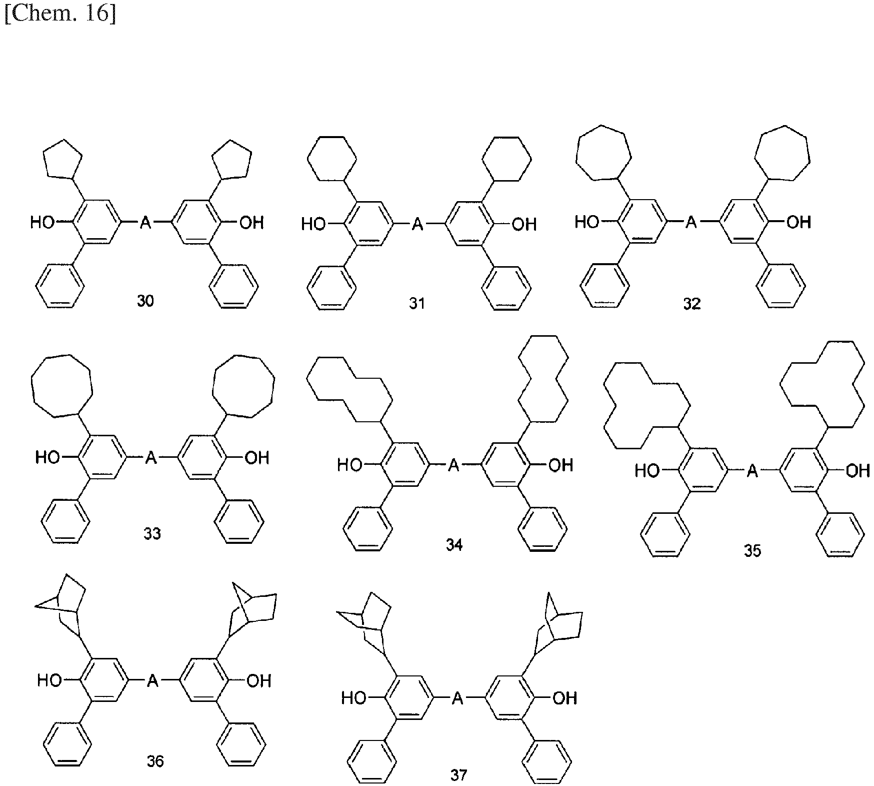

Each of the compounds represented by the above formulae (1) to (4) can be synthesized by known synthetic methods or is commonly available.

2-Cyclohexyl-6-phenylphenol that is one of the ligands of the aluminum catalyst in the present invention is a precursor of 2,6-diphenylphenol that has been conventionally used and can be produced easily and inexpensively in the presence of an acidic catalyst (JP-A-2009-269868).

The aluminum catalyst in the present invention is obtained by reacting at least one selected from the aluminum compounds represented by the above general formulae (1) and (2) with at least one selected from the hydroxy compounds represented by the general formulae (3) and (4).

At that time, at least one selected from the hydroxy compounds represented by the general formulae (3) and (4) is preferably reacted in a proportion (aluminum atom:compound molar ratio) of preferably from 1.0 to 5 equivalents, and more preferably from 1.4 to 3.5 equivalents, to at least one selected from aluminum compounds represented by the general formulae (1) and (2).

The reaction can be carried out in an inert gas atmosphere or in the presence of an inert solvent.

As the inert gas, it is preferable to use nitrogen, argon, or another rare gas, for example.

Examples of the inert solvent include an aliphatic hydrocarbon (such as hexane, heptane and octane), an alicyclic hydrocarbon (such as cyclohexane and methylcyclohexane), an aromatic hydrocarbon (such as benzene, toluene and xylene), an ether (such as diethyl ether, diisopropyl ether, dimethoxyethane, methyl tert-butyl ether, tetrahydrofuran, dioxane and dioxolane), a halogenated hydrocarbon (such as dichloromethane, dichloroethane and chlorobenzene), and the like. Of those, the preferred solvent is an organic solvent such as toluene and heptane. Those solvents are preferably used in the form of previously dried one or an anhydrous solvent.

The amount of the solvent to be used is preferably from 1 to 10,000 times and more preferably from 20 to 400 times, based on the volume of the hydroxy compound.

The reaction temperature is preferably from about -60 to 100°C, more preferably from about -30 to 50°C, and particularly preferably from about -10 to 30°C. The reaction is conducted for preferably from about 0.25 to 30 hours, and more preferably from about 0.5 to 10 hours, while maintaining the above temperature. Thus, the aluminum catalyst can be smoothly produced.

The aluminum catalyst in the present invention has the excellent effect as a catalyst when conducting an intramolecular reaction, particularly an intramolecular cyclization reaction.

The aluminum catalyst in the present invention can be used as a catalyst when the reaction of the cyclization reaction of citronellal in a racemic form or an optical active form is conducted to synthesize isopulegol in a racemic form or an optical active form.

A method for producing isopulegol by selectively cyclizing citronellal using the aluminum catalyst in the present invention is conducted by the reaction shown in the following reaction scheme.

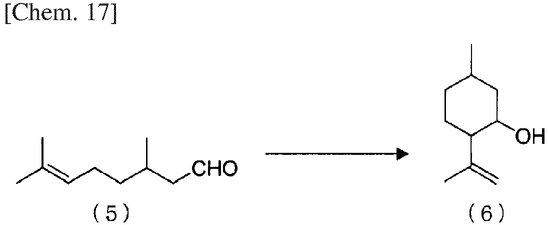

Furthermore, a method for producing optically active isopulegol by selectively cyclizing optically active citronellal using the aluminum catalyst in the present invention is conducted by the reaction shown in the following reaction scheme.

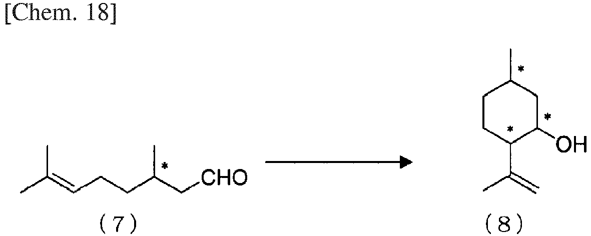

In the above reaction scheme, the "aluminum catalyst" has the same meaning as the aluminum catalyst in the present invention. Furthermore, in the general formulae (7) and (8), * means an asymmetric carbon atom.

Namely, in the above reaction schemes, isopulegol represented by the general formula (6) or (8) is formed by selectively cyclizing citronellal represented by the general formula (5) or (7) in the presence of the aluminum catalyst in the present invention.

As the citronellal which is a raw material, commercially available products can be used directly or after distillation by a common method.

The amount of the aluminum catalyst to be used in the cyclization reaction of citronellal in the present invention is preferably in the range of from about 0.05 to 10% by mole, and more preferably in the range of from about 0.1 to 3% by mole, based on the citronellal.

With regard to the aluminum catalyst to be used in the cyclization reaction of citronellal in the present invention, similar results can be obtained by any method of a) a method of previously mixing at least one selected from the alkylaluminum compound represented by the general formula (1) and the hydridoaluminum compound represented by the general formula (2) with at least one selected from hydroxy compounds represented by the general formulae (3) and (4) in the reaction system to prepare an aluminum catalyst, and then adding citronellal into the reaction system, and b) a method of individually adding an aluminum catalyst prepared by previously mixing at least one selected from the alkylaluminum compound and the hydridoaluminum compound with at least one selected from the hydroxy compounds, and citronellal, respectively, during the cyclization reaction.

The temperature of the cyclization reaction of citronellal is preferably in the range of from about -60 to 60°C, more preferably in the range of from about -30 to 40°C, and particularly preferably from about -20 to 20°C. By conducting the reaction for from about 0.25 to 30 hours, and more preferably from about 0.5 to 20 hours while maintaining the above temperature, isopulegol represented by the general formula (6) or (8) can be smoothly obtained.

The cyclization reaction of citronellal in the present invention can be conducted in the absence of a solvent, in the presence of an inert solvent or in an inert gas atmosphere.

The solvent to be used is not particularly limited so long as it is a solvent that does not remarkably disturb the reaction. Examples thereof include an aliphatic hydrocarbon (such as hexane, heptane and octane), an alicyclic hydrocarbon (such as cyclohexane and methylcyclohexane), an aromatic hydrocarbon (such as benzene, toluene and xylene), an ether (such as diethyl ether, diisopropyl ether, dimethoxyethane, methyl tert-butyl ether, tetrahydrofuran, dioxane and dioxolane), a halogenated hydrocarbon (such as dichloromethane, dichloroethane and chlorobenzene), and the like. Of these, the preferred solvent is an organic solvent such as toluene or heptane. Those solvents are preferably used in the form of previously dried one or an anhydrous solvent.

The amount of the solvent to be used is preferably from about 0 to 20 times, and more preferably from 0.5 to 7 times, based on the volume of the citronellal.

When conducting the cyclization reaction, an additive may be added. Specific examples of the additive include a mineral acid (such as hydrochloric acid and sulfuric acid), an organic acid and an ester compound thereof (such as formic acid, acetic acid, pyruvic acid, propionic acid, citronellylic acid, geranylic acid and nerylic acid or an alkyl/aryl ester thereof), an aldehyde other than citronellal (such as chloral, acetaldehyde, p-bromobenzaldehyde and ethyl glyoxylate), an organic acid anhydride (such as acetic anhydride, propionic anhydride, decanoic anhydride, maleic anhydride, citronellylic anhydride, succinic anhydride or pivaloic anhydride), a ketone (such as peuluoroacetone and 1,1,1-trifluoroacetone), an acid halide (such as acetyl chloride, propionyl chloride and decanoyl chloride), a vinyl ether (such as methyl vinyl ether and ethyl vinyl ether), and an epoxy compound (such as α-pinene oxide, isobutylene oxide and isopulegol oxide).

After the aluminum catalyst is prepared, the cyclization reaction of citronellal can be conducted by adding the acid and the ester compound thereof, the aldehyde other than citronellal, the acid anhydride, the ketone, the acid halide, the vinyl ether, or the epoxy compound to the catalyst layer or the citronellal layer.

The cyclization reaction is preferably conducted in an inert gas atmosphere such as nitrogen gas or argon gas for smooth progress of the cyclization reaction.

After completion of the reaction, the general post-treatment can be conducted. The isopulegol represented by the general formula (6) or (8) is purified by simply conducting the treatment by distillation without conducting cryogenic separation, and thus, isopulegol having a high purity can be obtained.

Furthermore, the residue after the distillation treatment is generally subjected to the usual treatment with an acid or an alkali, thereby removing impurities containing aluminum or the like, and after that, it is subjected to crystallization or the like. As a result, the hydroxy compound can be reutilized as a ligand.

On the other hand, in the organoaluminum compound in the present invention, an aluminum catalyst which is hard to be soluble in a solvent is removed by filtration of formed isopulegol after completion of the reaction, and it can be directly used in the subsequent reaction.

The ligand of all of the organoaluminum compounds is recovered after deactivation of catalyst, and then it can again be reutilized as the catalyst.

### Examples

The present invention is described in detail below by reference to Examples and Comparative Examples, but the present invention is not construed as being limited thereto, and may be modified as long as it does not deviate the scope of the present invention.

Measurement of products in Synthesis Examples and Examples was conducted using the following instruments and apparatus.
Nuclear Magnetic Resonance Spectrum (¹H-NMR): Oxford 300 MHz, FT-NMR, (300 MHz, solvent CDCl₃) (manufactured by Varian)
Gas chromatograph: GC-2010 Gas Chromatograph, manufactured by Shimadzu Corporation
Measurement of addition rate: DB-WAX (0.25 mmx30 m), manufactured by Agilent
Measurement of optical purity: beta-DEX-225 (0.25 mmx30 m), manufactured by Supelco
Detector: FID
Optical purity of each citronellal used in the present invention is as follows.
d-Citronellal: 97.8% e.e.
1-Citronellal: 96.6% e.e.

### (Example 1) Preparation of Aluminum Catalyst and Synthesis of 1-Isopulegol

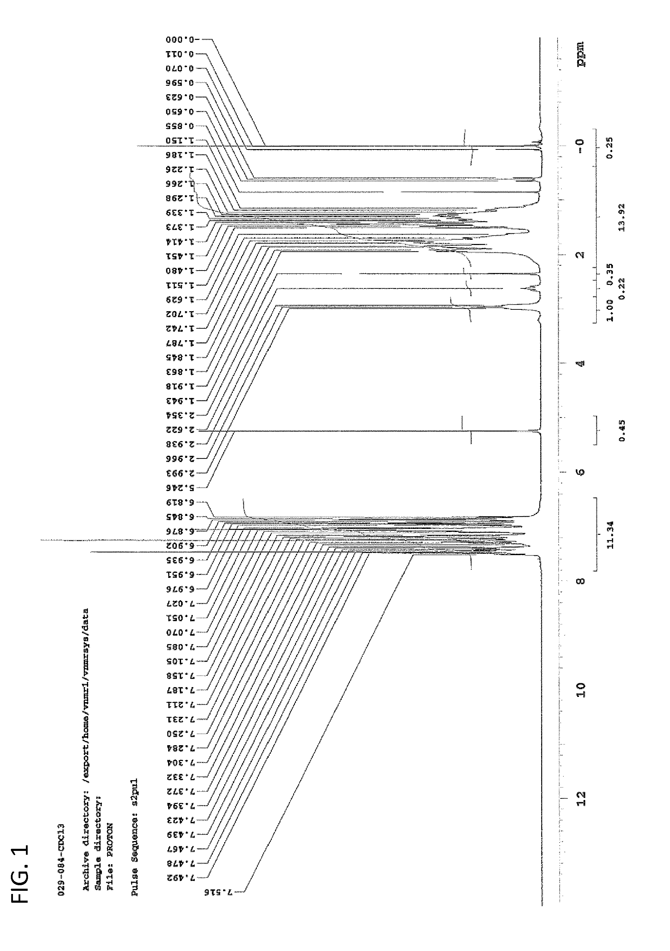

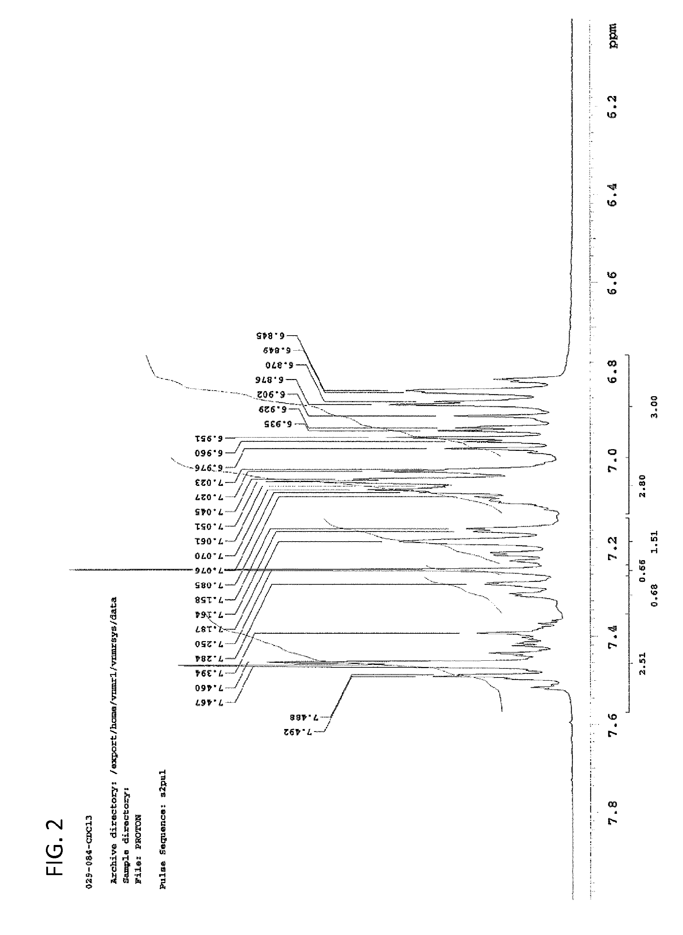

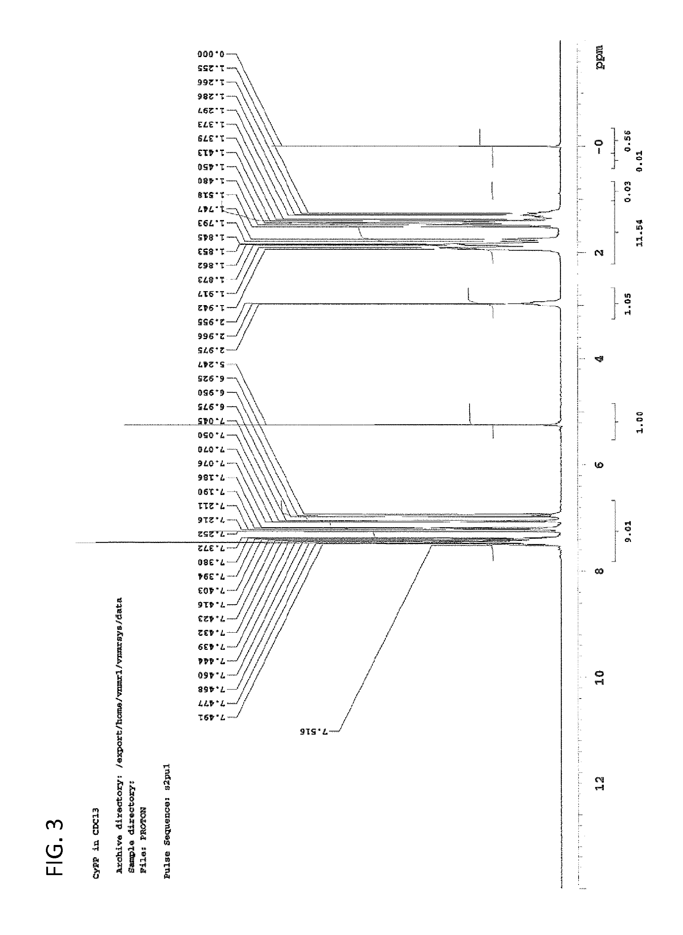

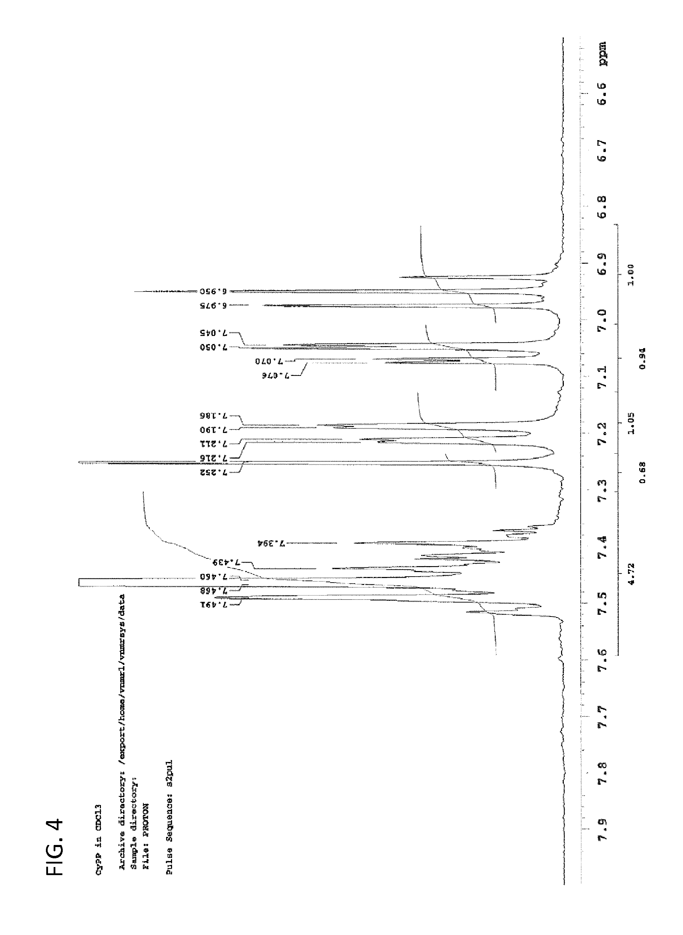

Under a nitrogen atmosphere, 0.34 g of 2-cyclohexyl-6-phenylphenol (1.36 mmol, manufactured by Sanko Co., Ltd. or synthesized according to the method described in JP-A-2009-269868 (the same shall apply hereinafter)) was placed in a 200 ml reaction flask. After nitrogen substitution, 4.9 ml of toluene and 0.39 ml (0.389 mmol) of a triethylaluminum-toluene solution (1.0 mol/L) were sequentially added thereto, and the resulting mixture was stirred at room temperature for 2 hours. Then, the solvent was distilled away to obtain 0.40 g of a colorless to light orange amorphous yellow solid. The solid obtained was dried by concentration under reduced pressure, and a ¹H-NMR spectrum thereof measured was shown in FIG. 1 and an enlarged view thereof at low magnetic field side is shown in FIG. 2. Also, a ¹H-NMR spectrum of 2-cyclohexyl-6-phenylphenol was shown in FIG. 3 and an enlarged view thereof at low magnetic field side is shown in FIG. 4.

Then, 234 mg of the solid obtained above was added to 2.00 g (13 mmol) of d-citronellal cooled to -15 to -10°C, followed by stirring at 0 to 5°C for 1 hour. After completion of the reaction, 2 ml of water and 2 ml of toluene were added thereto, and an organic layer was analyzed with gas chromatography. As a result, substrate conversion was 99.1 %, 1-isopulegol selectivity was 96.6%, and the ratio of 1-isopulegol to the other isomers was 99.5:0.5.

### (Example 2) Synthesis of 1-Isopulegol

In a 50 ml Schlenk flask, 344 mg (1.4 mmol) of 2-cyclohexyl-6-phenylphenol was placed. After nitrogen substitution, 1.6 ml of toluene and 0.4 ml (0.40 mmol) of a triethylaluminum-toluene solution (1.0 mol/L) were sequentially added thereto, and the resulting mixture was stirred at room temperature for 2 hours, thereby obtaining a catalyst solution. After the catalyst solution obtained was cooled to -15 to -10°C, 2.00 g (13 mmol) of d-citronellal was added dropwise, followed by stirring at 0 to 5°C for 1 hour. After completion of the reaction, 2 ml of water was added thereto, and an organic layer was analyzed with gas chromatography. As a result, substrate conversion was 99.8%, 1-isopulegol selectivity was 86.3%, and the ratio of 1-isopulegol to the other isomers was 99.6:0.4.

### (Example 3) Synthesis of d-Isopulegol

In a 50 ml Schlenk tube, 344 mg (1.4 mmol) of 2-cyclohexyl-6-phenylphenol was placed. After nitrogen substitution, 1.6 ml of toluene and 0.4 ml (0.40 mmol) of a triethylaluminum-toluene solution (1.0 mol/L) were sequentially added thereto, and the resulting mixture was stirred at room temperature for 2 hours, thereby obtaining a catalyst solution. After the catalyst solution obtained was cooled to -15 to -10°C, 2.00 g (13 mmol) of 1-citronellal was added dropwise, followed by stirring at 0 to 5°C for 1 hour. After completion of the reaction, 2 ml of water was added thereto, and an organic layer was analyzed with gas chromatography. As a result, substrate conversion was 99.2%, d-isopulegol selectivity was 82.1%, and the ratio of d-isopulegol to the other isomers was 99.3:0.7.

### (Example 4 and Comparative Example 1) Synthesis of d-Isopulegol (Comparison of Reaction Rate)

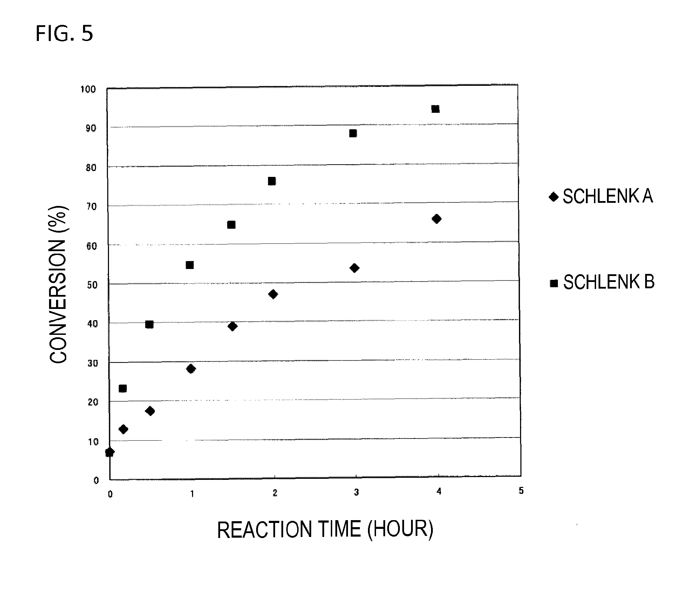

Two 50 ml Schlenk tubes were prepared, and 837 mg of 2,6-diphenylphenol (3.3 mmol, manufactured by Aldrich) was placed in one tube (Comparative Example 1, hereinafter referred to as Schlenk A) and 858 mg (3.3 mmol) of 2-cyclohexyl-6-phenylphenol was placed in the other tube (Example 4, hereinafter referred to as Schlenk B). After nitrogen substitution, 4.7 ml of toluene and 0.96 ml (0.96 mmol) of a triethylaluminum-toluene solution (1.0 mol/L) were sequentially added thereto, and the resulting mixture was stirred at room temperature for 2 hours, thereby obtaining a catalyst solution. After the catalyst solution obtained was cooled to -20°C, 5.00 g (32 mmol) of 1-citronellal was added dropwise to each tube, followed by stirring at -20°C for 3 hours. During reaction and aging, 0.1 ml of the solution in each Schlenk was sampled at every predetermined time, 1 ml of toluene and 1 ml of water were added thereto to terminate the reaction, and an organic layer was analyzed with gas chromatography. Reaction conversion in Schlenk A and Schlenk B was shown in FIG. 5.

### (Examples 5 to 10) Synthesis of 1-Isopulegol by Aluminum Catalyst

Results using various phenols as hydroxy compounds are shown in Table 1. With regard to reaction conditions, each of the phenols was placed in a 50 ml Schlenk tube in an amount of 1.7 mmol for Examples 5 to 9 and in an amount of 0.87 mmol for Example 10. After nitrogen substitution, 3 ml of toluene in total as a solvent and 0.58 ml (0.58 mmol) of a triethylaluminum-toluene solution were sequentially added thereto, and the resulting mixture was stirred at room temperature for 2 hours, thereby obtaining a catalyst solution. After the catalyst solution was cooled to -10°C, 3.0 g (19 mmol) of d-citronellal was added dropwise, followed by stirring for 1 hour. After completion of the reaction, 2 ml of water was added thereto, and an organic layer was analyzed with gas chromatography.

For Examples 5 to 10, each of the phenols was synthesized in the same manner as in the case of 2-cyclohexyl-6-phenylphenol according to the method described in JP-A-2009-269868.

In Table 1, conversion represents the conversion of citronellal, isopulegol selectivity represents the selectivity of the reacted citronellal to isopulegol, n-isopulegol selectivity represents the selectivity of n-isopulegol in the isopulegol formed, and ester selectivity represents the selectivity of citronellal to a dimerized ester of citronellal (citronellyl citronellate).

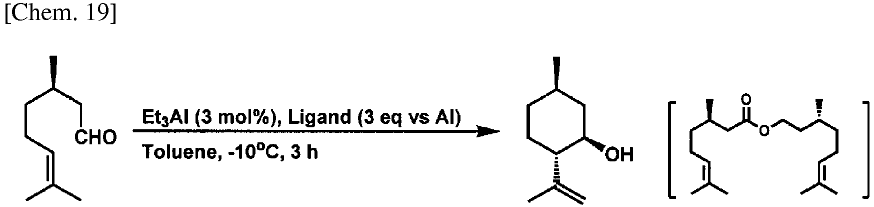

**[Table 1]**

| Ex. | Ligand | Conversion (%) | Isopulegol selectivity (%) | n-Selectivity (%) | Ester selectivity (%) |
|---|---|---|---|---|---|
| 5 | 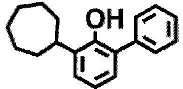 | 99.2 | 97.1 | 99.4 | 2.1 |
| 6 | 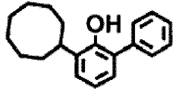 | 98.2 | 93.4 | 99.0 | 4.0 |
| 7 | 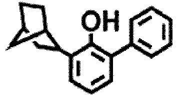 | 99.4 | 98.0 | 99.4 | 0.3 |
| 8 | 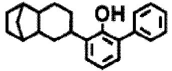 | 98.3 | 89.9 | 98.4 | 5.9 |
| 9 | 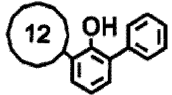 | 99.8 | 95.1 | 98.9 | 1.4 |
| 10 | 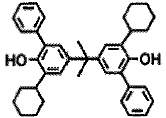 | 99.1 | 95.9 | 99.4 | 1.9 |

### (Examples 11 to 17) Synthesis of 1-Isopulegol by Aluminum Catalyst

In a 50 ml Schlenk tube, 269 mg (1.1 mmol) of 2-cyclohexyl-6-phenylphenol was placed. After nitrogen substitution, 4.7 ml of toluene and 0.3 ml (0.32 mmol) of a triethylaluminum-toluene solution (1.0 mol/L) were sequentially added thereto, and the resulting mixture was stirred at room temperature for 2 hours, thereby obtaining a catalyst solution. After the catalyst solution obtained was cooled to -15 to -10°C, 5.00 g (32 mmol) of d-citronellal containing 0.5% by weight of an additive added thereto was added dropwise, followed by stirring for 3 hours. After completion of the reaction, 2 ml of water was added thereto, and an organic layer was analyzed with gas chromatography. Results are shown in Table 2.

In Table 2, conversion represents the conversion of citronellal, isopulegol selectivity represents the selectivity of the reacted citronellal to isopulegol, n-isopulegol selectivity represents the selectivity of n-isopulegol in the isopulegol formed, and ester selectivity represents the selectivity of citronellal to a dimerized ester of citronellal (citronellyl citronellate).

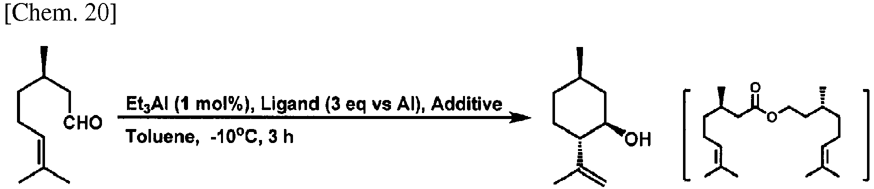

**[Table 2]**

| Ex. | Additive | Conversion (%) | Isopulegol selectivity (%) | n-Selectivity (%) | Ester selectivity (%) |
|---|---|---|---|---|---|
| 11 | Acetic anhydride | 99.5 | 96.9 | 99.6 | 0.3 |
| 12 | Citronellic acid | 99.5 | 94.6 | 99.5 | 0.6 |
| 13 | Monometyl itaconate | 42.6 | 89.4 | 99.0 | 4.0 |
| 14 | Ethyl glyoxylate 40wt% to 50wt% polymer toluene solution | 62.9 | 92.7 | 99.3 | 3.5 |
| 15 | α-Pinene oxide | 99.2 | 96.2 | 99.3 | 0.9 |
| 16 | Isobutylene oxide | 97.9 | 97.0 | 99.7 | 0.4 |

### (Comparative Examples 2 to 19) Synthesis of 1-Isopulegol by Aluminum Catalyst

Results using various phenols as hydroxy compounds are shown in Tables 3 and 4. With regard to reaction conditions, each of the phenols was placed in a 50 ml Schlenk tube in a predetermined amount (2.0 mmol). After nitrogen substitution, 3 ml of toluene in total as a solvent and 0.58 ml (0.58 mmol) of a triethylaluminum-toluene solution were sequentially added thereto, and the resulting mixture was stirred at room temperature for 2 hours, thereby obtaining a catalyst solution. After the catalyst solution was cooled to predetermined temperature, 3.0 g (19 mmol) of d-citronellal was added dropwise, followed by stirring at predetermined temperature for 1 hour. After completion of the reaction, 2 ml of water was added thereto, and an organic layer was analyzed with gas chromatography.

The phenols were those manufactured by Aldrich for Comparative Examples 2 to 16, 18 and 19 and one manufactured by Bepharm for Comparative Example 17.

In Tables 3 and 4, conversion represents the conversion of citronellal, isopulegol selectivity represents the selectivity of the reacted citronellal to isopulegol, n-isopulegol selectivity represents the selectivity of n-isopulegol in the isopulegol formed, and ester selectivity represents the selectivity of citronellal to a dimerized ester of citronellal (citronellyl citronellate).

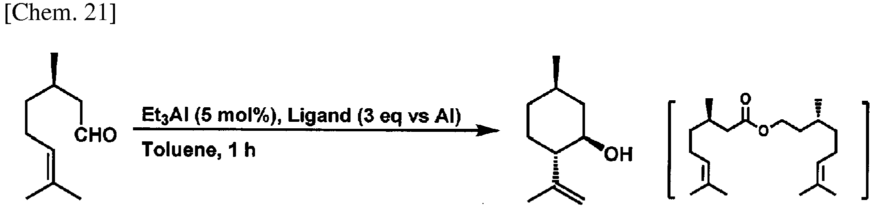

**[Table 3]**

| Comp. Ex. | Ligand | Temperature (°C) | Conversion (%) | Isopulegol selectivity (%) | n-Selectivity (%) | Ester selectivity (%) |
|---|---|---|---|---|---|---|
| 2 | 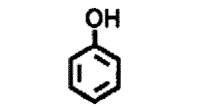 | 0 | 54.9 | 12.9 | 92.3 | 81 |
| 3 | 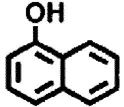 | 0 | 33.8 | 68.3 | 74.7 | 25.9 |
| 4 | 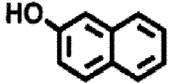 | 0 | 29.8 | 37.7 | 87.7 | 52.1 |
| 5 | 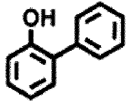 | 0 | 97 | 21.9 | 90.9 | 75.2 |
| 6 | 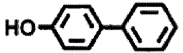 | 0 | 52.7 | 19.2 | 92.3 | 74.9 |
| 7 | 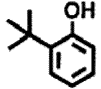 | 0 | 74.3 | 10.5 | 89.5 | 70.9 |
| 8 | 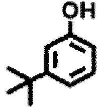 | 25 | 11.7 | 69.7 | 87.7 | 11.5 |
| 9 | 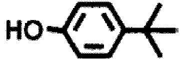 | 25 | 10.5 | 91.6 | 78.6 | 4.7 |
| 10 | 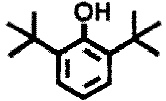 | 0 | 85.5 | trace | - | 90.9 |

**[Table 4]**

| Comp. Ex. | Ligand | Temperature (°C) | Conversion (%) | Isopulegol selectivity (%) | n-Selectivity (%) | Ester selectivity (%) |
|---|---|---|---|---|---|---|
| 11 | 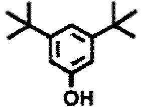 | 25 | 35.5 | 19.4 | 93.2 | 62.8 |
| 12 | 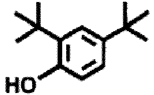 | 0 | 61.6 | 4.6 | 87.1 | 92.1 |
| 13 | 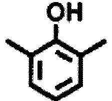 | 0 | 88.4 | 18.7 | 85.3 | 74.4 |
| 14 | 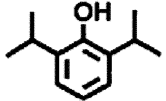 | 0 | 76.7 | 9.4 | 91.8 | 87.3 |
| 15 | 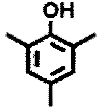 | 0 | 95.5 | 6.1 | 88.8 | 84.6 |
| 16 | 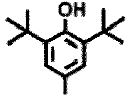 | 0 | 87 | trace | - | 89.9 |
| 17 | 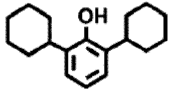 | -10 | 3.2 | 80.1 | 80.4 | 0.0 |
| 18 | 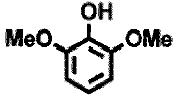 | 25 | trace | - | - | - |
| 19 | 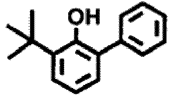 | -10 | 97.6 | 0.86 | 87.8 | 85.2 |

While the invention has been described in detail and with reference to specific embodiments thereof, it will be apparent to one skilled in the art that various changes and modifications can be made therein without departing from the spirit and scope thereof. The present application is based on Japanese Patent Application No. 2012-251301 filed on November 15, 2012, the contents of which are incorporated herein by reference.

## Claims

1. An aluminum catalyst obtained by reacting at least one compound of an alkylaluminum compound represented by the following general formula (1) and a hydridoaluminum compound represented by the following general formula (2) with a hydroxy compound represented by the following general formula (3):
AlHₘ(L_{g})₃₋ₘ (1)
wherein Al is aluminum; Lg is a branched, linear or cyclic alkyl group having from 1 to 8 carbon atoms; and m is an integer of 0 to 3;
MAlH₄ (2)
wherein Al is aluminum; and M is lithium, sodium or potassium; 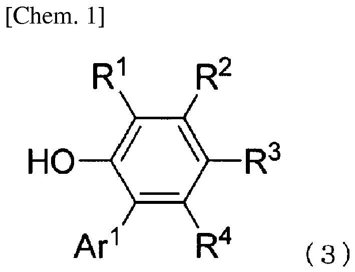 wherein Ar¹ is an aryl group having from 6 to 15 carbon atoms, which may have a substituent, or a heteroaryl group having from 2 to 15 carbon atoms, which may have a substituent;
R¹ is a cyclic alkyl group having from 5 to 12 carbon atoms, which may have a substituent; R², R³ and R⁴ each independently are a hydrogen atom, an alkyl group having from 1 to 8 carbon atoms, a cyclic alkyl group having from 5 to 12 carbon atoms, a perfluoroalkyl group having from 1 to 4 carbon atoms, an alkoxy group having from 1 to 8 carbon atoms, an aralkyl group having from 7 to 12 carbon atoms, which may have a substituent, a halogen atom, an organosilyl group, an aryl group having from 6 to 15 carbon atoms, which may have a substituent, a dialkylamino group having from 2 to 8 carbon atoms, a thioalkoxy group having from 1 to 4 carbon atoms, a nitro group or a polymer chain; and R² and R³, or R³ and R⁴ may each independently be combined with each other to form a condensed benzene ring, a condensed substituted-benzene ring, a trimethylene group, a tetramethylene group, a pentamethylene group, a methylenedioxy group, an ethylenedioxy group or a trimethylenedioxy group.

2. An aluminum catalyst obtained by reacting at least one compound of an alkylaluminum compound represented by the following general formula (1) and a hydridoaluminum compound represented by the following general formula (2) with a hydroxy compound represented by the following general formula (4):
AlHₘ(Lg)₃₋ₘ (1)
wherein Al is aluminum; Lg is a branched, linear or cyclic alkyl group having from 1 to 8 carbon atoms; and m is an integer of 0 to 3;
MAlH₄ (2)
wherein Al is aluminum; and M is lithium, sodium or potassium; 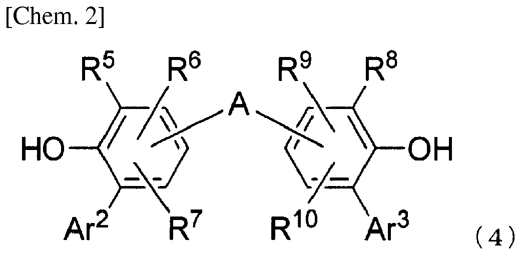 wherein Ar² and Ar³ each independently are an aryl group having from 6 to 15 carbon atoms, which may have a substituent, or a heteroaryl group having from 2 to 15 carbon atoms, which may have a substituent;
R⁵ and R⁸ each independently are a cyclic alkyl group having from 5 to 12 carbon atoms, which may have a substituent; R⁶, R⁷, R⁹ and R¹⁰ each independently are a hydrogen atom, an alkyl group having from 1 to 8 carbon atoms, a cyclic alkyl group having from 5 to 12 carbon atoms, a perfluoroalkyl group having from 1 to 4 carbon atoms, an alkoxy group having from 1 to 8 carbon atoms, an aralkyl group having from 7 to 12 carbon atoms, which may have a substituent, a halogen atom, an organosilyl group, an aryl group having from 6 to 15 carbon atoms, which may have a substituent, a dialkylamino group having from 2 to 8 carbon atoms, a thioalkoxy group having from 1 to 4 carbon atoms, a nitro group or a polymer chain; and R⁶ and R⁷, or R⁹ and R¹⁰ may each independently be combined with each other to form a condensed benzene ring, a condensed substituted-benzene ring, a trimethylene group, a tetramethylene group, a pentamethylene group, a methylenedioxy group, an ethylenedioxy group or a trimethylenedioxy group; and one or more of R⁶ or R⁷, and R⁹ or R¹⁰ may be combined with A to form an aromatic ring or a non-aromatic ring;
A is (i) a single bond, (ii) a linear, branched or cyclic alkylene group having from 1 to 25 carbon atoms, which may have one or more of a substituent and an unsaturated bond, (iii) an arylene group having from 6 to 15 carbon atoms, which may have a substituent, (iv) a heteroarylene group having from 2 to 15 carbon atoms, which may have a substituent, or (v) a functional group or a hetero element, which is selected from the group consisting of -O-, -S-, -N(R¹¹)-, -S(O)-, -C(O)-, -S(O)₂-, -P(R¹¹)-, -(R¹¹)P(O)- and -Si(R¹²R¹³)-, wherein R¹¹ to R¹³ each independently are an alkyl group having from 1 to 6 carbon atoms, a cyclic alkyl group having from 5 to 8 carbon atoms, an aralkyl group having from 7 to 12 carbon atoms, which may have a substituent, or an aryl group having from 6 to 10 carbon atoms, which may have a substituent.

3. A method for producing isopulegol represented by the general formula (6), comprising a step of selectively cyclizing citronellal represented by the general formula (5) using the aluminum catalyst according to claim 1 or the aluminum catalyst according to claim 2.

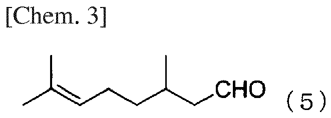

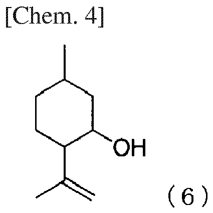

4. A method for producing optically active isopulegol represented by the general formula (8), comprising a step of selectively cyclizing citronellal represented by the general formula (7) using the aluminum catalyst according to claim 1 or the aluminum catalyst according to claim 2. 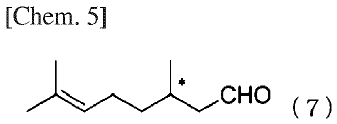 wherein * means an asymmetric carbon atom; 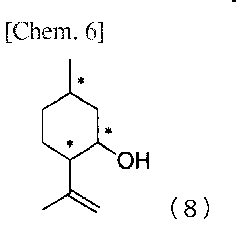 wherein * means an asymmetric carbon atom.

5. A method for producing isopulegol represented by the general formula (6), comprising a step of selectively cyclizing citronellal represented by the general formula (5) in the presence of at least one compound of the following compounds I and II using the aluminum catalyst according to claim 1 or the aluminum catalyst according to claim 2:
I. at least one acid;
II. at least one compound selected from the group consisting of an aldehyde other than citronellal, an acid anhydride, a ketone, an acid halide, an epoxy compound and a vinyl ether.

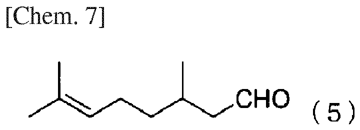

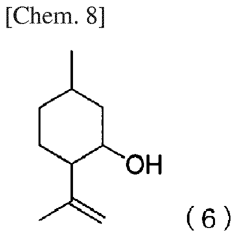

6. A method for producing optically active isopulegol represented by the general formula (8), comprising a step of selectively cyclizing citronellal represented by the general formula (7) in the presence of at least one compound of the following compounds I and II using the aluminum catalyst according to claim 1 or the aluminum catalyst according to claim 2:
I. at least one acid;
II. at least one compound selected from the group consisting of an aldehyde other than citronellal, an acid anhydride, a ketone, an acid halide, an epoxy compound and a vinyl ether. 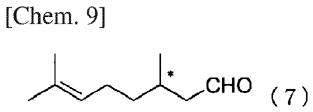 wherein * means an asymmetric carbon atom; 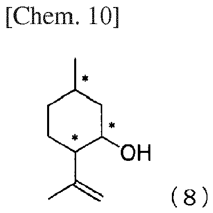 wherein * means an asymmetric carbon atom.
